# EUROPEAN PATENT APPLICATION

(11) **EP 3 696 560 A1**
(43) Date of publication of application: **19.08.2020**
(21) Application number: 19157099.3
(22) Date of filing: 14.02.2019
(51) Int. Cl.: G01R 33/34, G01R 33/341, G01R 33/3415, A61N 5/00, G01R 33/28, G01R 33/36, G01R 33/48, G01R 33/54

(54) **ADHESIVE MAGNETIC RESONANCE IMAGING ANTENNA**

(71) Applicant: Koninklijke Philips N.V., 5656 AG Eindhoven (NL)
(72) Inventor: LEUSSLER, Christoph, 5656 AE Eindhoven (NL); HELLE, Michael Günter, 5656 AE Eindhoven (NL); VOGTMEIER, Gereon, 5656 AE Eindhoven (NL)
(74) Representative: de Haan, Poul Erik

(57) **Abstract**

The invention provides for a medical instrument (100, 400, 700, 900, 1100, 1500) comprising an adhesive magnetic resonance imaging antenna (102). The adhesive magnetic resonance imaging antenna comprises: a flexible dielectric layer (104), a flexible cover layer (106), a flexible magnetic resonance imaging coil element (108), an adhesive pad (110) attached to the flexible dielectric layer, and an antenna connector (116) configured for providing a radio frequency connection to the flexible magnetic resonance imaging coil element. The flexible magnetic resonance imaging coil element is formed between the flexible dielectric layer and the flexible cover layer. The flexible dielectric layer and the flexible cover layer electrically insulate the flexible magnetic resonance imaging coil element. The adhesive pad is configured for adhering to a surface.

## Description

### FIELD OF THE INVENTION

The invention relates to magnetic resonance imaging, in particular to antennas or coils used for magnetic resonance imaging.

### BACKGROUND OF THE INVENTION

A large static magnetic field is used by Magnetic Resonance Imaging (MRI) scanners to align the nuclear spins of atoms as part of the procedure for producing images within the body of a patient. This large static magnetic field is referred to as the B0 field or the main magnetic field. When nuclear spins are aligned in the B0 field a radio-frequency (RF) field, often referred to as the B1 field, maybe used to move spins to an angle relative to the B0 Field. The spins can be further manipulated and spatially differentiated using gradient magnetic fields in one or more directions. Individual spins that have rotated relative to the B0 field begin to process and produce a radio frequency signal. A magnetic resonance imaging antenna or coil may be used to receive the collective RF signal from many spins. The recorded (digitized) values of this collective RF signal are called magnetic resonance imaging data herein and may be used to reconstruct a magnetic resonance image.

United States patent application publication US 2006/0279284 A1 discloses a radio frequency magnetic coil coupled to a wireless communication circuit. The wireless communication circuit allows control or monitoring of individual channels or other functions of a coil. It discloses that the coil can be configured for placement on a patient while in an examining room or dressing room and worn, or carried on the body, into the MR magnet for an MR scan. The coil, in one example, includes an adhesive coated surface that bonds to a surface such as a skin, textile, or other surface. Individual elements of the coil can be fabricated on a flexible substrate using semiconductor fabrication technology. In one example, the present subject matter includes a wireless coil in the form of a bandage.

### SUMMARY OF THE INVENTION

A difficulty with the wireless coil in the form of a bandage of US 2006/0279284 A1 is that the wireless communication circuit would be expensive and produce toxic electronic waste. The bandage would likely be used once and then thrown away. Embodiments of the invention may provide for reduced cost and electronic waste by separating the coil into multiple components. One embodiment may consist of an adhesive magnetic resonance imaging antenna that has a flexible magnetic resonance imaging coil element between a flexible dielectric layer and a flexible cover layer. An adhesive pad attached to the flexible dielectric layer may be suitable for affixing to a skin or other surface. A separate antenna connector then provides an RF connection to the flexible magnetic resonance imaging coil element. The adhesive magnetic resonance imaging coil may then be attached to a subject or other object without, for example, preamplifier or other electronics.

The invention provides for a medical instrument which comprises an adhesive magnetic resonance imaging antenna. The adhesive magnetic resonance imaging antenna comprises a flexible dielectric layer, a flexible cover layer, and a flexible magnetic resonance imaging coil element. The flexible magnetic resonance imaging coil element is formed between the flexible dielectric layer and the flexible cover layer. The flexible dielectric layer and the flexible cover layer electrically insulate the flexible magnetic resonance imaging coil element. The adhesive magnetic resonance imaging antenna further comprises an adhesive pad attached to the flexible dielectric layer. The adhesive pad is configured for or suitable for adhering to a surface such as a skin surface. The adhesive magnetic resonance imaging antenna further comprises an antenna connector configured for providing a radio-frequency connection to the flexible magnetic resonance imaging coil element. This embodiment may be beneficial because it may provide for a subject-adapted magnetic resonance imaging antenna that provides a high image quality at a reduced cost. The invention may also provide for the adhesive magnetic resonance imaging antenna separate of any other components of a medical instrument.

The flexible dielectric layer and the flexible cover layer may be any type of flexible dielectric which is also useful for assembling electronic connections. For example, the materials used for making so called flex circuits may be used. In some examples the flexible dielectric layer and/or the flexible cover layer are any one of or a combination of polyimide, peek, Teflon or transparent conductive polyester film.

In another embodiment the adhesive magnetic resonance imaging antenna is free of active electronic components. This may be beneficial because the inclusion of active electronic components on the adhesive magnetic resonance imaging antenna may increase its cost. The elimination of active electronic components may also make the adhesive magnetic resonance imaging antenna more compatible with magnetic resonance guided radio therapy.

In another embodiment the adhesive magnetic resonance imaging antenna comprises an overload element. For example, a capacitor, diode or fuse may be used and incorporated into the adhesive magnetic resonance imaging antenna to protect a subject or subject from an over-voltage of the adhesive magnetic resonance imaging antenna. For example, if the adhesive magnetic resonance imaging antenna were attached to a subject and then it was not attached to the accompanying electronics, there may be a voltage which builds up during a magnetic resonance imaging protocol. The use of a capacitor, diode or fuse may reduce or eliminate the risk to a subject in this situation.

In another embodiment the adhesive magnetic resonance imaging antenna is free of discreet electronic components. Being free of discreet electronic components may also have the benefit of making the adhesive magnetic resonance imaging antenna more radiation therapy friendly or compatible.

In another embodiment the adhesive layer is configured for being controllably released from the surface. Controllably released as used herein means that the adhesive pad's connection to a surface can be weakened using a non-mechanical method.

In another embodiment the adhesive pad comprises an adhesive. The adhesive is a temperature releasable adhesive. In this embodiment a temperature heating device may be used to controllably release the adhesive magnetic resonance imaging antenna.

In another embodiment the adhesive pad comprises an adhesive that is an ultraviolet light releasable adhesive. The flexible dielectric layer and flexible cover layer are transparent to ultraviolet light. This embodiment may be beneficial because an ultraviolet light can be used to weaken the adhesive such that it can be removed with a minimal amount of discomfort to a subject.

In another embodiment the adhesive pad comprises an adhesive that is alcohol releasable. The flexible dielectric layer and the flexible cover layer comprise perforations configured for transporting alcohol. The flexible dielectric layer and the flexible cover layer are configured for sealing the flexible magnetic resonance imaging coil element from the alcohol. This embodiment may be beneficial because it may provide for a straight forward and inexpensive means to remove the adhesive magnetic resonance imaging antenna from a subject painlessly.

In another embodiment the adhesive magnetic resonance imaging antenna comprises an ECG electrode. The antenna connector is further configured for providing an electrical connection to the ECG electrode. This embodiment may be beneficial because particularly for doing magnetic resonance imaging protocols of the heart a single adhesive magnetic resonance imaging antenna can be used for both ECG measurements and for performing the magnetic resonance imaging protocol.

In another embodiment the antenna connector is formed from multiple conductors formed on any one of the following: the flexible dielectric layer and the flexible cover layer.

In another embodiment the antenna connector is configured for forming a radio-frequency connector with a pre-amplifier connector of a magnetic resonance antenna pre-amplifier using any one of the following: a clamping attachment; with an adhesive attachment; and with a socket and plug attachment.

In another embodiment the antenna connector comprises multiple capsules. The multiple capsules are configured to rupture during the application of a pre-amplifier connector to a magnetic resonance antenna pre-amplifier to the antenna connector. The multiple capsules are configured to provide any one of the following: to provide a radio-frequency electrical connection between the antenna connector and the pre-amplifier connector, provide electrical insulation, provide waterproofing, and combinations thereof. This embodiment maybe beneficial because it may provide for a low cost means of connecting the pre-amplifier connector with the antenna connector. The use of the multiple capsules for instance may be used to provide for electrical connections which are both electrically isolated and waterproof before and after joining of the connectors.

In another embodiment the medical instrument further comprises a magnetic resonance antenna pre-amplifier. The magnetic resonance antenna pre-amplifier is configured for digitizing radio-frequency signals received by the adhesive magnetic resonance imaging antenna. The invention may in some aspects provide for an assembly or kit formed from the combination of the magnetic resonance antenna pre-amplifier and the adhesive magnetic resonance imaging antenna.

The magnetic resonance antenna pre-amplifier may be reusable and may also comprise all of the active electronic components. This may be very beneficial in reducing the cost of the adhesive magnetic resonance imaging antenna.

In another embodiment, the flexible magnetic resonance imaging antenna may comprise an optical position indicator such as a visible mark or indicator which may be used to determine a location and orientation of the flexible magnetic resonance imaging antenna. This may for example be used by an automated positioning system to assist in the positioning of the flexible magnetic resonance imaging antenna.

In another embodiment, the medical instrument comprises an automated positioning system comprising a computational system and a camera. The computational system is configured to: receive camera data from the camera, recognize an antenna position using the camera data, recognize a target location on the subject using the camera data, and provide placement instructions using the antenna position and the target location. The antenna position may for example be located using a neural network and/or a visible indicator. The target location may for example be determined by fitting the camera data to a subject model or by using the neural network.

In another embodiment the medical instrument comprises a magnetic resonance imaging system. The magnetic resonance imaging system is configured for receiving the digitized magnetic resonance imaging data from the magnetic resonance antenna pre-amplifier. This embodiment may be beneficial because the adhesive magnetic resonance imaging antenna in combination with the magnetic resonance imaging system may provide a very flexible, high quality and low cost receive coil.

In another embodiment the medical instrument further comprises a radiotherapy system configured for generating a radiation beam. This embodiment may be beneficial because the adhesive magnetic resonance imaging antenna may produce a smaller portion of the radiation beam than a conventional magnetic resonance imaging antenna.

In some examples the adhesive magnetic resonance imaging antenna may have a transmission line to facilitate the moving of the electronics away from the radiation beam.

In another embodiment the medical instrument further comprises a magnetic resonance imaging antenna array formed from multiple adhesive magnetic resonance imaging antennas attached to a surface. This embodiment may be beneficial because it may enable the customization of a magnetic resonance imaging antenna array for individual subjects.

In another embodiment the adhesive magnetic resonance imaging antenna is configured to be movable when attached to a skin surface surrounding a joint. This embodiment may be particularly beneficial because it may facilitate the measurement of a joint in multiple positions using magnetic resonance imaging.

The facilitating of the movement of the adhesive magnetic resonance imaging antenna when attached to a joint may be performed in several different ways. For example, the magnetic resonance imaging antenna may be formed as a strip similar to an adhesive bandage which can then be readily attached to a joint. In other examples there may be portions of the adhesive magnetic resonance imaging antenna that are cut away or have relief points in them to facilitate the bending of the adhesive magnetic resonance imaging antenna.

In another aspect the invention provides for a method of magnetic resonance imaging using a medical instrument that comprises a magnetic resonance imaging system. The method comprises applying at least one adhesive magnetic resonance imaging antenna to a surface of a subject or other object. The application of the adhesive magnetic resonance imaging antenna to the surface of the subject may comprise sub-steps. For example, a computer in combination with a camera could be used to recognize the position of the adhesive magnetic resonance imaging antenna and guide the user to place it properly.

The method further comprises connecting the antenna connector of each of the at least one adhesive magnetic resonance imaging antenna to a pre-amplifier connector of the pre-amplifier. The method further comprises connecting the pre-amplifier to the magnetic resonance imaging system. The method further comprises placing the subject in an imaging zone of the magnetic resonance imaging system. The method further comprises controlling the magnetic resonance imaging system with the pulse sequence commands. The method further comprises receiving digitized magnetic resonance imaging data from the magnetic resonance imaging antenna pre-amplifier during controlling the magnetic resonance imaging system with pulse sequence commands. The method further comprises reconstructing the magnetic resonance image from the digitized magnetic resonance imaging data.

In another embodiment one of the adhesive magnetic resonance imaging antennas is configured to be movable when attached to the skin surface surrounding a joint of the subject. The method further comprises repeating the method for different joint positions of the joint. This embodiment may be beneficial because it enables the acquisition of magnetic resonance images for different joint positions readily.

It is understood that one or more of the aforementioned embodiments of the invention may be combined as long as the combined embodiments are not mutually exclusive.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, microcode, etc.) or an embodiment combining software and hardware aspects that may all generally be referred to herein as a "circuit," "module" or "system." Furthermore, aspects of the present invention may take the form of a computer program product embodied in one or more computer readable medium(s) having computer executable code embodied thereon.

Any combination of one or more computer readable medium(s) maybe utilized. The computer readable medium may be a computer readable signal medium or a computer readable storage medium. A 'computer-readable storage medium' as used herein encompasses any tangible storage medium which may store instructions which are executable by a processor of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the processor of the computing device. Examples of computer-readable storage media include, but are not limited to: a floppy disk, a magnetic hard disk drive, a solid-state hard disk, flash memory, a USB thumb drive, Random Access Memory (RAM), Read Only Memory (ROM), an optical disk, a magneto-optical disk, and the register file of the processor. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example, a data may be retrieved over a modem, over the internet, or over a local area network. Computer executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

'Computer memory' or 'memory' is an example of a computer-readable storage medium. Computer memory is any memory which is directly accessible to a processor. 'Computer storage' or 'storage' is a further example of a computer-readable storage medium. Computer storage is any non-volatile computer-readable storage medium. In some embodiments computer storage may also be computer memory or vice versa.

A 'processor' as used herein encompasses an electronic component which is able to execute a program or machine executable instruction or computer executable code. References to the computing device comprising "a processor" should be interpreted as possibly containing more than one processor or processing core. The processor may for instance be a multi-core processor. A processor may also refer to a collection of processors within a single computer system or distributed amongst multiple computer systems. The term computing device should also be interpreted to possibly refer to a collection or network of computing devices each comprising a processor or processors. The computer executable code may be executed by multiple processors that may be within the same computing device or which may even be distributed across multiple computing devices.

Computer executable code may comprise machine executable instructions or a program which causes a processor to perform an aspect of the present invention. Computer executable code for carrying out operations for aspects of the present invention may be written in any combination of one or more programming languages, including an object oriented programming language such as Java, Smalltalk, C++ or the like and conventional procedural programming languages, such as the "C" programming language or similar programming languages and compiled into machine executable instructions. In some instances the computer executable code may be in the form of a high level language or in a pre-compiled form and be used in conjunction with an interpreter which generates the machine executable instructions on the fly.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer executable code when applicable. It is further under stood that, when not mutually exclusive, combinations of blocks in different flowcharts, illustrations, and/or block diagrams may be combined. These computer program instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks.

These computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks. A 'user interface' as used herein is an interface which allows a user or operator to interact with a computer or computer system. A 'user interface' may also be referred to as a 'human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.

A 'hardware interface' as used herein encompasses an interface which enables the processor of a computer system to interact with and/or control an external computing device and/or apparatus. A hardware interface may allow a processor to send control signals or instructions to an external computing device and/or apparatus. A hardware interface may also enable a processor to exchange data with an external computing device and/or apparatus. Examples of a hardware interface include, but are not limited to: a universal serial bus, IEEE 1394 port, parallel port, IEEE 1284 port, serial port, RS-232 port, IEEE-488 port, Bluetooth connection, Wireless local area network connection, TCP/IP connection, Ethernet connection, control voltage interface, MIDI interface, analog input interface, and digital input interface.

A 'display' or 'display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen, Cathode ray tube (CRT), Storage tube, Bi-stable display, Electronic paper, Vector display, Flat panel display, Vacuum fluorescent display (VF), Light-emitting diode (LED) displays, Electroluminescent display (ELD), Plasma display panels (PDP), Liquid crystal display (LCD), Organic light-emitting diode displays (OLED), a projector, and Head-mounted display.

Magnetic Resonance Imaging (MRI) data is defined herein as being the recorded measurements of radio frequency signals emitted by atomic spins using the antenna of a Magnetic resonance apparatus during a magnetic resonance imaging scan. A Magnetic Resonance Imaging (MRI) image or MR image is defined herein as being the reconstructed two- or three-dimensional visualization of anatomic data contained within the magnetic resonance imaging data. This visualization can be performed using a computer.

### BRIEF DESCRIPTION OF THE DRAWINGS

In the following preferred embodiments of the invention will be described, by way of example only, and with reference to the drawings in which:
Fig. 1 illustrates an example of an adhesive magnetic resonance imaging antenna;
Fig. 2 illustrates a cross sectional view of the adhesive magnetic resonance imaging antenna of Fig. 1;
Fig. 3 illustrates a further example of an adhesive magnetic resonance imaging antenna;
Fig. 4 illustrates an example of a medical instrument;
Fig. 5 shows a flow chart which illustrates a method of operating the medical instrument of fig. 4;
Fig. 6 illustrates an example of an array of adhesive magnetic resonance imaging antennas;
Fig. 7 illustrates a further example of a medical instrument;
Fig. 8 shows a cross sectional view of the adhesive magnetic resonance imaging antenna depicted in Fig. 7;
Fig. 9 illustrates a further example of a medical instrument;
Fig. 10 illustrates a further example of an array of adhesive magnetic resonance imaging antennas;
Fig. 11 illustrates a further example of a medical instrument;
Fig. 12 illustrates the attachment of adhesive magnetic resonance imaging antennas to a radiation mask;
Fig. 13 illustrates a further example of an adhesive magnetic resonance imaging antenna;
Fig. 14 illustrates an example of an adhesive antenna connector 112; and
Fig. 15 illustrates a further example of a medical instrument.

### DETAILED DESCRIPTION OF THE EMBODIMENTS

Like numbered elements in these figures are either equivalent elements or perform the same function. Elements which have been discussed previously will not necessarily be discussed in later figures if the function is equivalent.

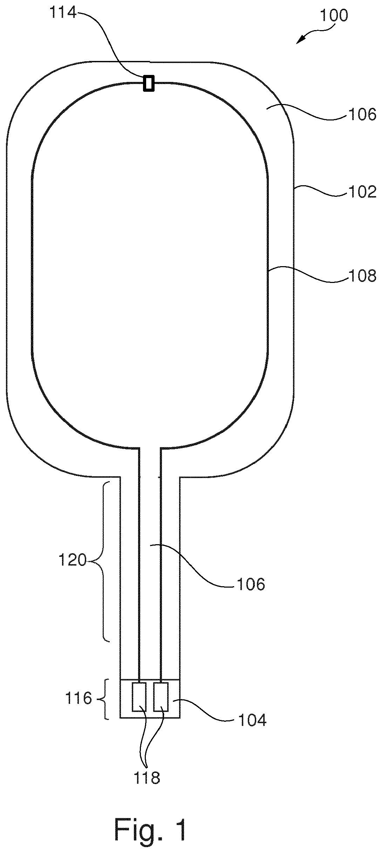

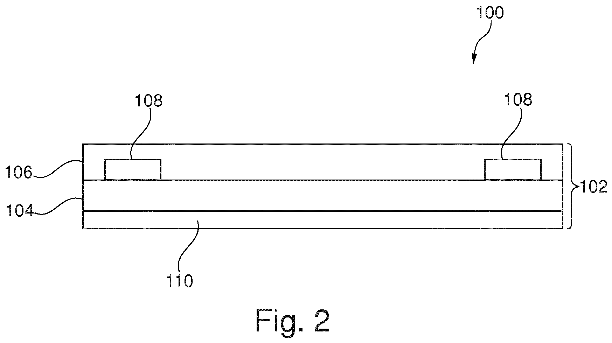

Figs. 1 and 2 show a view of a medical instrument 100 that comprises an adhesive magnetic resonance imaging antenna 102. Fig. 1 shows a top view and Fig. 2 shows a cross-sectional view. There is a flexible dielectric layer 104 and a flexible cover layer 106. Between the flexible dielectric layer 104 and the flexible cover layer 106 is a flexible magnetic resonance imaging coil element 108. For example, the flexible magnetic resonance imaging coil element 108 could be laminated between the flexible dielectric layer 104 and the flexible cover layer 106. On an outer surface of the flexible dielectric layer 104 is an adhesive pad 110. The adhesive pad 110 may be useful for attaching the adhesive magnetic resonance imaging antenna to various surfaces such as for example a skin surface of the subject or to other objects such as a radiation mask.

The adhesive magnetic resonance imaging antenna 102 is shown as further comprising an optional fuse component. The fuse component 114 may for example be a fuse which prevents there being an over-voltage or over-current condition on the flexible magnetic resonance imaging coil element 108. The fuse component 114 is distributed in series electrically with the flexible magnetic resonance imaging coil element 108. If the current through the fuse is too high it will break and provide a high series impedance.

The adhesive magnetic resonance imaging antenna 102 further comprises an antenna connector 116 which provides a radio-frequency connection to the flexible magnetic resonance imaging coil element 108. In Fig. 1 there is an optional transmission line 120 which forms a connection between the flexible magnetic resonance imaging coil element 108 and the antenna connector 116. In this example the antenna connector 116 is formed by several pad conductors 118 which are each connected to part of the transmission line 120. An effective electrical connection can be made to the flexible magnetic resonance imaging coil element 108 for example by using an electrical connector that clamps onto the antenna connector 116.

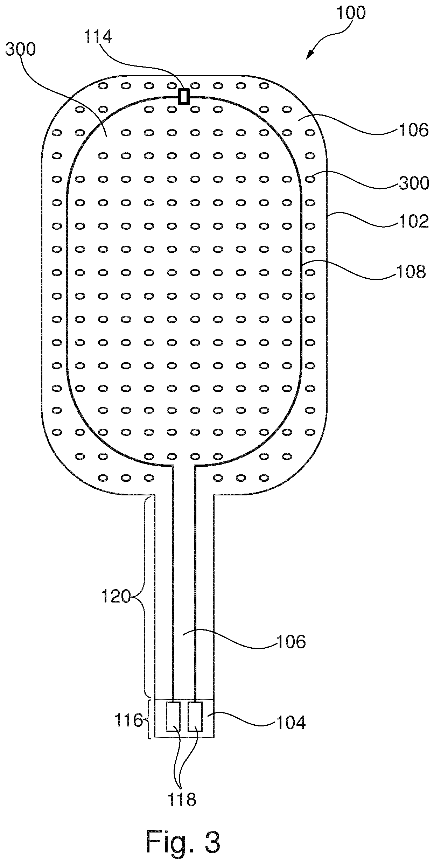

Fig. 3 shows a modification of the adhesive magnetic resonance imaging antenna 102 that is depicted in Figs. 1 and 2. In the example of Fig. 3 there have been a number of perforations 300 that have been punched through both the flexible dielectric layer 104 and the flexible cover layer 106. These perforations 300 may enable easy removal of the adhesive magnetic resonance imaging antenna 102. For example, the adhesive pad 110 may be constructed using an adhesive that is soluble or releases when in contact with alcohol. To remove the adhesive magnetic resonance imaging antenna 102 of Fig. 3 a healthcare professional can simply swab alcohol on the exposed surface. The alcohol then travels through the perforations and causes the adhesive pad 110 to release. It should be noted that the perforations 300 do not punch through the flexible magnetic resonance imaging coil element 108 and the flexible dielectric layer 104 and the flexible cover layer 106 seal the flexible magnetic resonance imaging coil element 108 from any alcohol.

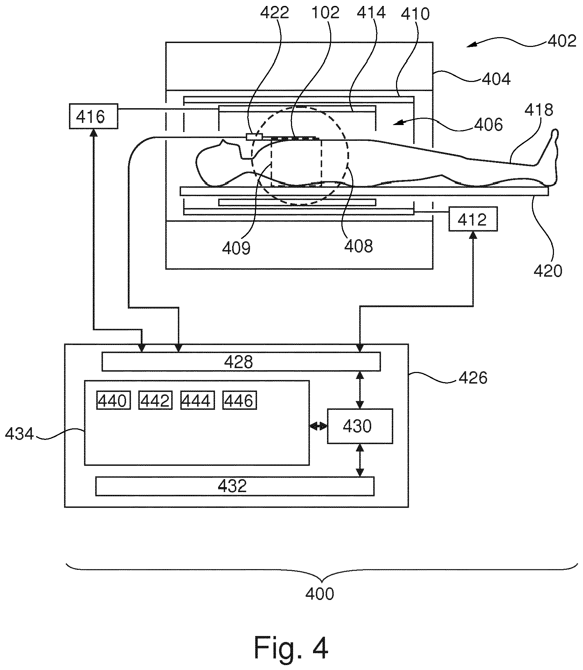

Fig. 4 illustrates a further example of a medical instrument 400. The medical instrument 400 in Fig. 4 comprises a magnetic resonance imaging system 402, a pre-amplifier 422 and an adhesive magnetic resonance imaging antenna 102.

The magnetic resonance imaging system 402 comprise a magnet 404. The magnet 404 is a superconducting cylindrical type magnet with a bore 406 through it. The use of different types of magnets is also possible; for instance it is also possible to use both a split cylindrical magnet and a so called open magnet. A split cylindrical magnet is similar to a standard cylindrical magnet, except that the cryostat has been split into two sections to allow access to the iso-plane of the magnet, such magnets may for instance be used in conjunction with charged particle beam therapy. An open magnet has two magnet sections, one above the other with a space in-between that is large enough to receive a subject: the arrangement of the two sections area similar to that of a Helmholtz coil. Open magnets are popular, because the subject is less confined. Inside the cryostat of the cylindrical magnet there is a collection of superconducting coils. Within the bore 406 of the cylindrical magnet 404 there is an imaging zone 408 where the magnetic field is strong and uniform enough to perform magnetic resonance imaging. A region of interest 409 is shown within the imaging zone 408. The magnetic resonance imaging data that is acquired typically acquried for the region of interest. A subject 418 is shown as being supported by a subject support 420 such that at least a portion of the subject 418 is within the imaging zone 408 and the region of interest 409.

Within the bore 406 of the magnet there is also a set of magnetic field gradient coils 410 which is used for acquisition of preliminary magnetic resonance imaging data to spatially encode magnetic spins within the imaging zone 408 of the magnet 404. The magnetic field gradient coils 410 connected to a magnetic field gradient coil power supply 412. The magnetic field gradient coils 410 are intended to be representative. Typically magnetic field gradient coils 410 contain three separate sets of coils for spatially encoding in three orthogonal spatial directions. A magnetic field gradient power supply supplies current to the magnetic field gradient coils. The current supplied to the magnetic field gradient coils 410 is controlled as a function of time and may be ramped or pulsed.

Adjacent to the imaging zone 408 is a radio-frequency coil 414 or body coil for manipulating the orientations of magnetic spins within the imaging zone 408 and for possibly also receiving radio transmissions from spins also within the imaging zone 408. The radio frequency antenna may contain multiple coil elements. The radio frequency antenna may also be referred to as a channel or antenna. The radio-frequency coil 414 is connected to a radio frequency transceiver 416. The radio-frequency coil 414 and radio frequency transceiver 416 may be replaced by separate transmit and receive coils and a separate transmitter and receiver. It is understood that the radio-frequency coil 414 and the radio frequency transceiver 416 are representative. The radio-frequency coil 414 may also represent also represent a dedicated transmit antenna and a dedicated receive antenna. Likewise the transceiver 416 may also represent a separate transmitter and receivers. The radio-frequency coil 414 may also have multiple receive/transmit elements and the radio frequency transceiver 416 may have multiple receive/transmit channels. For example if a parallel imaging technique such as SENSE is performed, the radio-frequency could 414 will have multiple coil elements.

The adhesive magnetic resonance imaging antenna 102 is shown as being affixed to a surface of the subject 418. The adhesive magnetic resonance imaging antenna 102 is then connected to the pre-amplifier 422 which is able to communicate via the hardware interface 428 of the computer system 426. For example, there may be a fiber optic link which connects the pre-amplifier 422 to a computer system 426. The adhesive magnetic resonance imaging antenna 102 is able to receive magnetic resonance signals independently from any receive channels formed by the body coil 414 and the transceiver 416. The adhesive magnetic resonance imaging antenna 102 may therefore be used to supplement existing receive channels of the magnetic resonance imaging system 402.

The transceiver 416, the gradient controller 412, and the preamplifier 422 are shown as being connected to a hardware interface 428 of a computer system 426. The computer system further comprises a processor 430 that is in communication with the hardware system 428, a memory 434, and a user interface 432. The memory 434 may be any combination of memory which is accessible to the processor 430. This may include such things as main memory, cached memory, and also non-volatile memory such as flash RAM, hard drives, or other storage devices. In some examples the memory 434 maybe considered to be a non-transitory computer-readable medium.

The memory 434 is shown as containing machine-executable instructions 440. The machine-executable instructions 440 enable the processor 430 to control the operation and function of the medical instrument 400. The machine-executable instructions 140 may also enable the processor 130 to perform various data analysis and calculation functions. The memory 434 is further shown as containing pulse sequence commands 442. The pulse sequence commands contain instructions or data which may be converted into instructions, which enable the processor 430 to control the magnetic resonance imaging system to acquire magnetic resonance imaging data 444. The memory 434 is shown as containing magnetic resonance imaging data 444 that has been acquired by controlling the magnetic resonance imaging system 402 with the pulse sequence commands 442. The memory 434 is further shown as containing a magnetic resonance image 446 that has been reconstructed from the magnetic resonance imaging data 444.

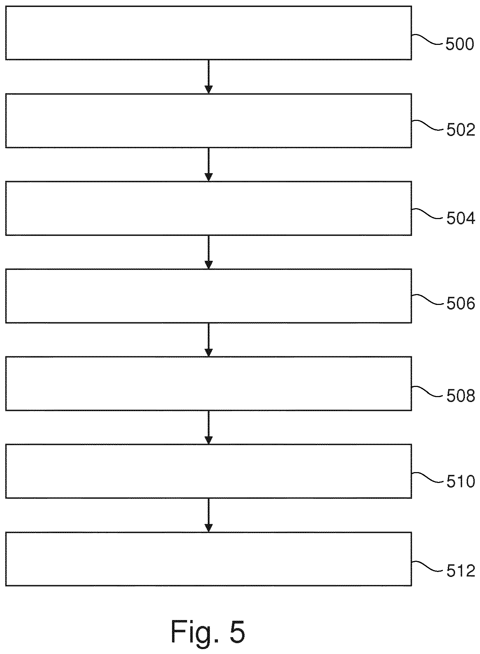

Fig. 5 shows a flowchart which illustrates a method of operating the medical instrument 400 of Fig. 4. First in step 500 the method comprises applying at least one adhesive magnetic resonance imaging antenna 102 to a skin surface of a subject 418. Then in step 502, the antenna connector of each of the at least one magnetic resonance imaging antenna is connected to the pre-amplifier connector of the pre-amplifier 422. Then in step 504, the pre-amplifier 422 is connected to the magnetic resonance imaging system 402. In this case the pre-amplifier 422 is connected to the hardware interface 428 of the computer system 426. Then in step 506, the subject 418 is placed in the imaging zone 408 of the magnetic resonance imaging system 402. In step 508, the magnetic resonance imaging system 402 is controlled with the pulse sequence commands 442. Next in step 510, the digitized magnetic resonance imaging data 444 is received from the magnetic resonance antenna pre-amplifier 422. Then in step 512, the magnetic resonance image 446 is reconstructed from the magnetic resonance imaging data 444.

The adhesive magnetic resonance imaging antenna 102 may also be referred to as an RF sticker coil, which comprises at least one RF coil, which can be directly glued to the subject's surface or on textile surface, coil frame holder or radiation mask. The coil comprises a metallic coil conductor inside an isolating layer. The outer surface of the insulating layer is covered with an adhesive surface. The RF sticker coil may provide for a subject adapted antenna configuration providing high image quality at low cost.

To date an increased workflow speed for preparation and scanning of a subject is often required. This enhances throughput and reduces cost. Therefore, the user would like to
- prevent using many dedicated coils for different organs ('one-stop-shop concept')
- optimize image quality for dedicated solutions
- configure and position of coils for dedicated applications
- provide magnetic resonance imaging coils for radiation therapy
- enhance organ specific imaging speed without interruption of the exam, repositioning and swap of RF-coils
- survey with the body coil and directly image with an appropriate dedicated coil array.
- position individual coils for a specific therapy.

Thin RF coils, which can be adhesively fixed on the body have a number of advantages for dedicated applications such as Radiation therapy and difficult dedicated applications such as the head neck region and carotids. Examples may have thin RF coils (disposable) of different size, which are connected to a reusable electronic unit (preamplifier, ADC detuning,...).

The proposed adhesive magnetic resonance imaging coils may be radiation transparent for radiotherapy. For example, the coil may be constructed so that it is free of lumped capacitors.

Due to the ultra-high input impedance, the coils can easily be connected to the human body, a coil frame, or a radiation mask for LINAC radiotherapy. Specialized software could assist the user to fix the coils to the object. SNR optimization could be achieved by locally adjusting the density of individual coil-elements and using element geometries adapted to a typical exam for that region of the body or organ.

Dedicated sticker coils may allow for high-quality images of moving joints (imaging of hand), whereby tendons and ligaments can be rendered moving in relation to bones and muscle, and could be useful in helping to guide surgery. This may be helpful for the design of prostheses and for diagnosing conditions such as carpal tunnel syndrome.

Problems or disadvantages which might be overcome by examples might include one or more of the following:
- Coils need to be configured for dedicated applications
- No solution for transparent disposable RF coils for radiotherapy
- RF coils are not radiation transparent because of capacitors.
- Dedicated coils do cover only limited subject size
- Moving joints SNR quality is low
- Different organ specific dedicated coils hamper workflow, are expensive and probably not applicable in one exam without interruption of the scanning process, repositioning of the subject and/or change of coils.
- Large body coils are not optimized for different body regions and do not provide the possibility of accelerated imaging.

The adhesive magnetic resonance imaging coil 102, or RF coil concept (Sticker Coil), which consists of RF coils, which can be directly glued or adhered to a subject surface or on textile surface, coil frame holder or radiation mask. The coils 102 comprises of a metallic coil conductor (108 flexible magnetic resonance imaging coil element) inside an isolating layer (flexible dielectric layer 104 and flexible cover layer 106). The outer surface of the insulating layer is covered with an adhesive surface (adhesive pad 110). The adhesive magnetic resonance imaging coil 102 may allow subject 418 adapted antenna configuration providing high image quality at low cost.

A connector interface (antenna connector 116) allows the adhesive coil to be connected to the MR scanner. The connecting interface is formed as a plug with integrated electronics (preamplifier, digital compression, detuning etc.) (422 preamplifier). The coil 102 can be a single coil or a coil array. When there is no connection to the coil, the coil is inherently safe, as the conduction structure does not resonate. Only with the connector plug, the coil is an antenna structure.

In one example, the adhesive magnetic resonance imaging antenna can be used to form any type of volume/array coil design.

In one example the adhesive coils are connected to a connector interface. The coils can have different size and are disposable.

In another example the coils can be reused by the application of a disposable adhesive sticker. The coil is connected to the sticker via clamps or Velcro tape.

The individual coils are affixed to the subject or other object using a software tool and a camera, which predicts to the operator the optimal location of the coil placement.

The adhesive magnetic resonance imaging antenna can have an integrated ECG sensor. The signal from the sensor is directly processed by the connecting coil interface.

A kit could be provided in which the individual adhesive magnetic resonance imaging antenna are stored and instruction on how to place them on the subject. For example, the instructions could be in the form of an algorithm or computer program product which locates the adhesive magnetic resonance imaging antenna and indicates their proper placement.

Individual coils (adhesive magnetic resonance imaging antennas) can be almost freely positioned on the body to generate a dedicated coil array. Markers or indicators could be located or displayed on the surface of the coil stickers as a help for the operator for optimal configuration.

As an alternative, coil structures could be printed on a foil (conductive ink) or even directly on the skin also using an isolating interface material that also could be printed on the skin to build a sandwich layer structure. The interface material cold be silicone like and/or have other bio compatible properties that ensure also later the removal from the skin. For longer time application biocompatible glue could be printed to fix the foil and/or the directly printed coil structure.

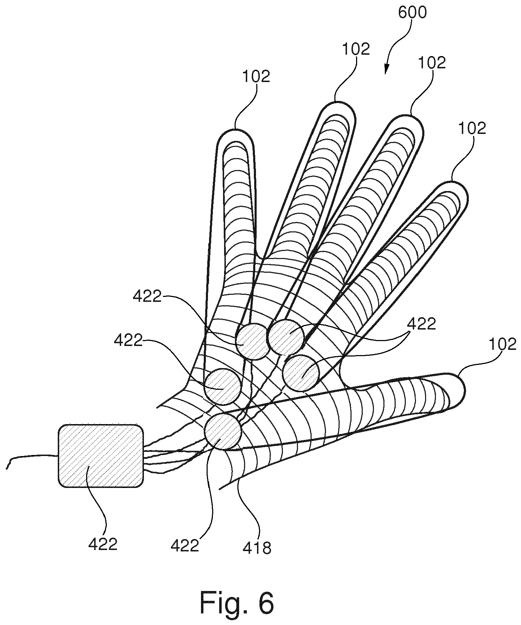

Fig. 6 illustrates the use of multiple adhesive magnetic resonance imaging antennas 102 on a hand of a subject 418. In this example there is an adhesive magnetic resonance imaging antenna 102 placed on each finger. At the end of the adhesive magnetic resonance imaging antenna 102 there is the pre-amplifier 422. In this example each of the pre-amplifiers 422 are shown as connecting to an additional pre-amplifier component 422'. However, in other examples the presence of the pre-amplifier component 422' is not necessary, there could just be the pre-amplifiers 422. Placing the adhesive magnetic resonance imaging antennas 102 on each of the fingers may enable the imaging of the hand in different positions. The multiple adhesive magnetic resonance imaging antennas 102 may also be referred to as configurable disposable sticker coils 102 for imaging of moving joints (hand, wrist). The sticker coils 102 are directly fixed on the joints and connected to an interface device connected to the MRI scanner.

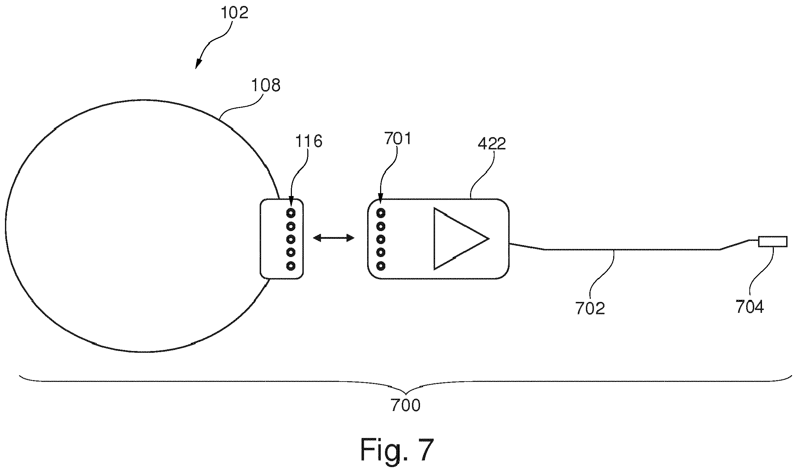

Fig. 7 illustrates a further example of a medical instrument 700. In this example the medical instrument 700 comprises an adhesive magnetic resonance imaging antenna 102 and a magnetic resonance antenna pre-amplifier 422. The features illustrated in Fig. 7 may be incorporated into Fig. 4. The adhesive magnetic resonance imaging antenna 102 has an antenna connector 116 that is configured for connecting to a pre-amplifier connector 701 of the magnetic resonance antenna pre-amplifier 422. The connectors 116 and 701 may represent various types of connectors such as sockets, an adhesive connector and a connector that is held together by a clamp. The magnetic resonance antenna pre-amplifier 422 is further shown as having a fiber optic 702 which connects to a fiber optic connector 704. The pre-amplifier 422 may for example digitize the magnetic resonance imaging data and transmit it to the computer via the fiber optic 702.

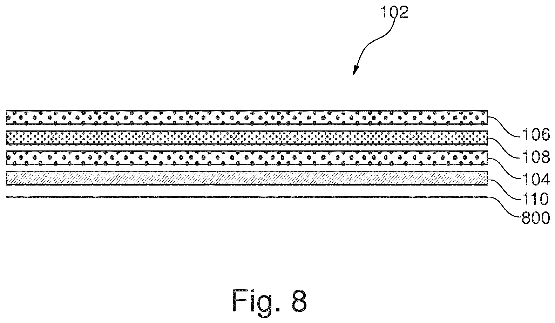

Fig. 8 shows layers which can be used to manufacture or build an adhesive magnetic resonance imaging antenna 102. As is illustrated further there is a top flexible cover layer 106 which may also be referred to as an isolation layer. There is the coil layer 108 which is the flexible magnetic resonance imaging coil element 108, then below this there is the flexible dielectric layer 104. This may also be referred to as an isolation layer. Attached to the flexible dielectric layer 104 is the adhesive pad 110. In this example there is a removable protective layer 800 that can be peeled off to expose the adhesive pad 110 before it is fixed to a surface.

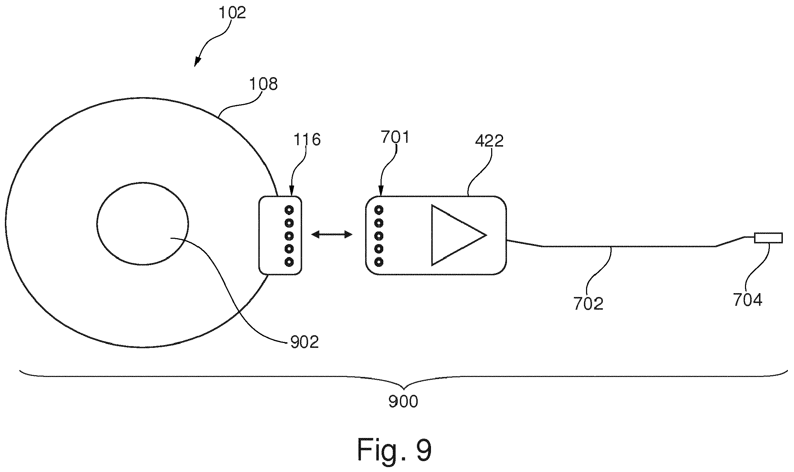

Fig. 9 illustrates a medical instrument 900 similar as to what was illustrated in Fig. 7. In this case the adhesive magnetic resonance imaging antenna 102 additionally comprises an ECG electrode 902. The ECG electrode 902 can be connected to the pre-amplifier and the pre-amplifier electronics 422 can be used to make the ECG measurements on a subject. This may for example be beneficial for several reasons. There are a fewer number of paths or sensors that need to be affixed to the subject and also, in the example illustrated in Fig. 9, the ECG electrode 902 has a fixed position with relation to the flexible magnetic resonance imaging coil element 108. This means that the manufacture of the adhesive magnetic resonance imaging antenna 102 with the ECG electrode 902 enables an optimum placement of the ECG electrode 902 so as to interfere with the recording of the magnetic resonance imaging data as little as possible.

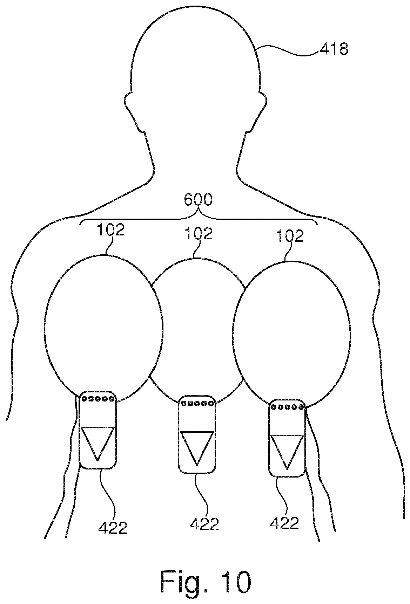

Fig. 10 illustrates the use of multiple adhesive magnetic resonance imaging antennas 102 to form a magnetic resonance imaging antenna array 600. To achieve this, three adhesive magnetic resonance imaging antennas 102 have been affixed to the chest region of the subject 418.

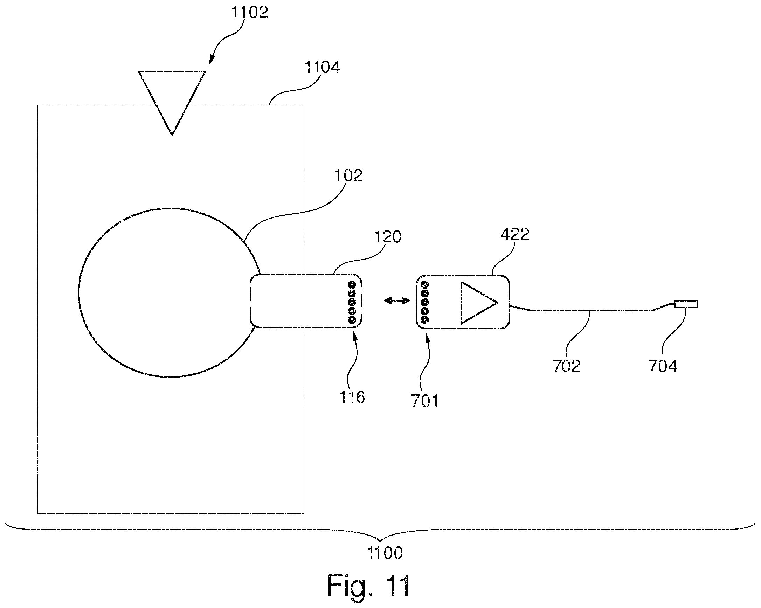

Fig. 11 illustrates a medical instrument 1100 that is similar to the medical instrument 700 of Fig. 7 except in this case the medical instrument 1100 additionally comprises a combined magnetic resonance imaging system and radiotherapy system 1102. The radiotherapy system could for example be a LINAC system. The box 1104 illustrates the region where there may be radiation or LINAC radiation. To move the active electronics of the pre-amplifier 422 out of the radiation area 1104 the adhesive magnetic resonance imaging antenna 102 comprises the transmission line 120. This enables moving components which may attenuate the radiation 1104 away from this region.

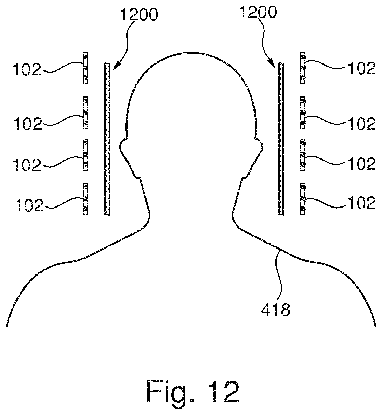

Fig. 12 illustrates a further application of the adhesive magnetic resonance imaging antenna 102. In this example the subject 418 has been placed with a radiation mask 1200 for performing radiation therapy. A radiation mask as used herein, is a mask which is used to precisely position the subject 418 for radiotherapy. One application is instead of placing the adhesive magnetic resonance imaging antennas 102 on the surface of the subject the adhesive magnetic resonance imaging antennas 102 are instead placed on the surface of the radiation mask. This essentially creates a custom magnetic resonance imaging array that is radiation compatible and facilitates the MRI guidance of radiotherapy.

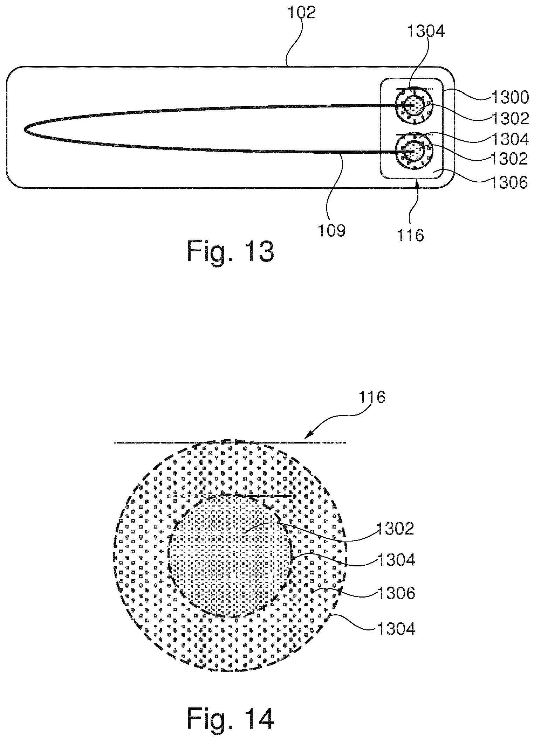

Fig. 13 is used to illustrate an alternative method of connecting the adhesive magnetic resonance imaging antenna 102 to the pre-amplifier. In this example the antenna connector 116 is a connection that forms using an adhesive. The coil connector 116 in this case is an adhesive connector 1300. The adhesive connector 1300 comprises two conductors 1302 which are connected to ends of the flexible magnetic resonance imaging coil element 108. Around the conductors 1302 is an insulator 1304. Surrounding both of these is then an adhesive 1306. The adhesive connector 1300 can be connected to a like designed connector on the magnetic resonance antenna pre-amplifier 422. Various types of adhesives could be used. There could be an adhesive with a debonding on demand functionality which allows selective operation bonding and debonding via temperature or for example ultraviolet light. These materials could be used to connect the coil embedded in a kind of plastic foil to the electronic connector foil. The adhesive would be located around the electrical contact that is either a kind of pad or in combination with a spring to ensure electrical contact during mechanical stress.

Fig. 14 shows an alternative embodiment of the adhesive connector. In this example the connector uses a number of bubbles filled with different types of functional materials being either the conductive material, isolating material, adhesive material, a combination of the conductive material and the adhesive material, and a combination of the isolating material and the adhesive material. If pressure is applied the bubble will collapse and a defined amount of adhesive is located around the bubble position defining the functional area of the electric contact the isolation area and/or the adhesive area. In Fig. 14 there are bubbles in the center which form the conductor 1302. Around the conductor 1302 is a layer of bubbles which when compressed form the insulator 1304. Surrounding this is then a further ring of bubbles which when compressed form the adhesive 1306. And again, there is an optional additional ring of the insulator material 1304.

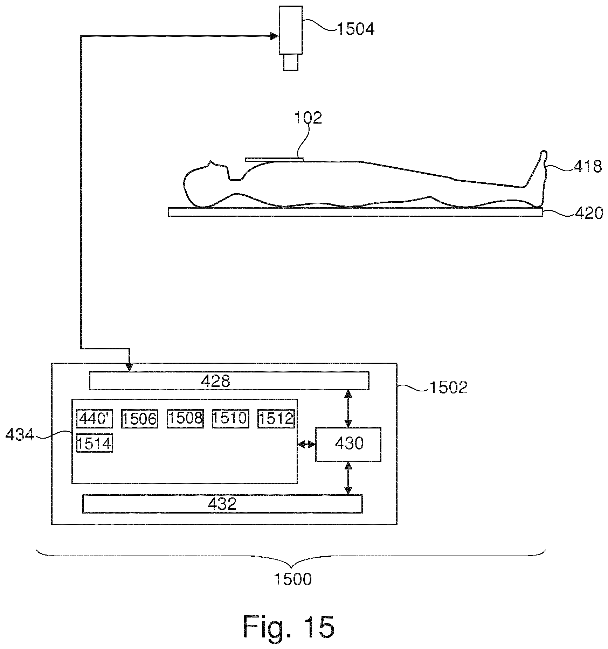

Fig. 15 shows a further example of a medical instrument 1500. In this example the medical instrument comprises a computational system 1502 and a camera system 1504. The computational system 1502 may be identical with the computer system 426 of Fig. 4 or it may be a separate system. The camera system 1504 is configured for imaging both the subject 418 and an adhesive magnetic resonance imaging antenna 102. The camera system outputs camera data 1506 to the computational system 1502. The memory 434 is shows as comprising machine executable instructions 440' and a positioning module 1508. The positioning module 1508 is able to identify an antenna position 1510 using the camera data 1506 as input. The positioning module 1508 is able to identify a target location 1512 on the subject using the camera data as input. The target location 1512 is a desired placement of the antenna 102 and provide placement instructions 1514 using the antenna position 1510 and the target location 1512. The positioning module 1508 may for example be a trained neural network and provide the placement instructions 1514 in one step. Alternatively, the target location may for example be determined by the positioning module 1508 by fitting the camera data 1506 to a subject model. The placement instructions 1514 could for example be displayed using the user interface 432.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measured cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

### LIST OF REFERENCE NUMERALS

- 100: medical instrument
- 102: adhesive magnetic resonance imaging antenna
- 104: flexible dielectric layer
- 106: flexible cover layer
- 108: flexible magnetic resonance imaging coil element
- 110: adhesive pad
- 112: antenna connector
- 114: fuse component
- 116: antenna connector
- 118: pad conductors
- 120: transmission line
- 300: perforation
- 400: medical instrument
- 402: magnetic resonance imaging system
- 404: magnet
- 406: bore of magnet
- 408: imaging zone
- 409: region of interest
- 410: magnetic field gradient coils
- 412: magnetic field gradient coil power supply
- 414: body coil
- 416: transceiver
- 418: subject
- 420: subject support
- 422: magnetic resonance antenna preamplifier
- 422': preamplifier component
- 426: computer system
- 428: hardware interface
- 430: processor
- 432: user interface
- 434: computer memory
- 440: machine executable instructions
- 440': machine executable instructions
- 442: pulse sequence commands
- 444: magnetic resonance imaing data
- 446: magetic resonance image
- 500: applying at least one adhesive magnetic resonance imaging antenna to a surface of a subject
- 502: connecting the antenna connector of each of the at least one adhesive magnetic resonance imaging antenna to a preamplifier connector of a preamplifier
- 504: connecting the preamplifier to the magnetic resonance imaging system
- 506: placing the subject in an imaging zone of the magnetic resonance imaging system
- 508: controlling the magnetic resonance imaging system with pulse sequence commands
- 510: receiving magnetic resonance imaging data from the magnetic resonance antenna preamplifier during controlling the magnetic resonance imaging system with the pulse sequence commands
- 512: reconstructing a magnetic resonance image from the magnetic resonance imaging data
- 600: magnetic resonance imaging antenna array
- 700: medical instrument
- 701: preamplifier connector
- 702: fiber optic
- 704: fiber optic connector
- 800: removable protective layer
- 900: medical instrument
- 902: ECG electrode
- 1100: medical instrument
- 1102: combined MRI and radio theraphy system
- 1104: radiation area
- 1200: radiation mask
- 1300: adhesive connector
- 1302: conductor
- 1304: insulator
- 1306: adhesive
- 1500: medical instrument
- 1502: computational system
- 1504: camera system
- 1506: camera data
- 1508: positioning module
- 1510: antenna position
- 1512: target location
- 1514: placement instructions

## Claims

1. A medical instrument (100, 400, 700, 900, 1100, 1500) comprising an adhesive magnetic resonance imaging antenna (102), wherein the adhesive magnetic resonance imaging antenna comprises:
- a flexible dielectric layer (104);
- a flexible cover layer (106);
- a flexible magnetic resonance imaging coil element (108), wherein the flexible magnetic resonance imaging coil element is formed between the flexible dielectric layer and the flexible cover layer, wherein flexible dielectric layer and the flexible cover layer electrically insulate the flexible magnetic resonance imaging coil element;
- an adhesive pad (110) attached to the flexible dielectric layer, wherein the adhesive pad is configured for adhering to a surface; and
- an antenna connector (116) configured for providing a radio frequency connection to the flexible magnetic resonance imaging coil element.

2. The medical instrument of claim 1, wherein the adhesive magnetic resonance imaging antenna is free of active electronic components.

3. The medical instrument of claim 1 or 2, wherein the adhesive layer is configured for being controllably released from the surface.

4. The medical instrument of any one of the preceding claims, wherein the adhesive pad comprises an adhesive, wherein any one of the following:
- the adhesive is a temperature releasable adhesive;
- the adhesive is an ultraviolet light releasable adhesive, wherein the flexible dielectric layer and the flexible cover layer are transparent to ultraviolet light;
- the adhesive is an alcohol releasable adhesive, wherein the flexible dielectric layer and the flexible cover layer comprise perforations (300) configured for transporting alcohol, wherein the flexible dielectric layer and the flexible cover layer are configured for sealing the flexible magnetic resonance imaging coil element from the alcohol.

5. The medical instrument of any one of the preceding claims, wherein the adhesive magnetic resonance imaging antenna comprises an ECG electrode (902), wherein the antenna connector is further configured for providing an electrical connection to the ECG electrode.

6. The medical instrument of any one of the preceding claims, wherein the medical instrument comprises a magnetic resonance imaging antenna array (600) formed from multiple adhesive magnetic resonance imaging antennas attached to a surface.

7. The medical instrument of any one of the preceding claims, wherein the adhesive magnetic resonance imaging antenna is configured to be flexible when attached to a skin surface surrounding a joint.

8. The medical instrument of any one of the preceding claims, wherein the antenna connector is formed from multiple conductors formed on any one of the following: the flexible dielectric layer and the flexible cover layer.

9. The medical instrument of claim 8, wherein the antenna connector is configured for forming a radio frequency connection with a preamplifier connector of a magnetic resonance antenna preamplifier using any one of the following:
- with a clamping attachment;
- with an adhesive attachment (1300); and
- with a socket and plug attachment.

10. The medical instrument of claim 8, wherein the antenna connector comprises multiple capsules, wherein the multiple capsules are configured to rupture during application of a preamplifier connector a magnetic resonance antenna preamplifier to the antenna connector, wherein the multiple capsules are configured to provide any one of the following: provide a radio frequency electrical connection (1302) between the antenna connector and the preamplifier connector, provide electrical insulation (1304), provide waterproofing (1304, 1306), and combinations thereof.

11. The medical instrument of claim 8, 9, or 10, wherein the medical instrument further comprises a magnetic resonance antenna preamplifier (422), wherein the magnetic resonance antenna preamplifier is configured for digitizing radiofrequency signals received by the adhesive magnetic resonance imaging antenna into magnetic resonance imaging data (444).

12. The medical instrument of claim 11, wherein the medical instrument comprises a magnetic resonance imaging system (402), wherein the magnetic resonance imaging system is configured for receiving the magnetic resonance imaging data from the magnetic resonance antenna preamplifier.

13. The medical instrument of claim 12, wherein the medical instrument further comprises a radiotherapy system (1102) configured for generating a radiation beam (1104).

14. The medical instrument (1500) of any one of the preceding claims, wherein the medical instrument further comprises a computational system (1502) and a camera system (1504), wherein the camera system is configured for providing camera data (1506) descriptive of a subject (418) and the adhesive magnetic resonance imaging antenna (102), wherein the computational system is configured for outputting placement instructions (1514) for the adhesive magnetic resonance imaging antenna on a surface of the subject (418) in response to receiving the camera data as input.

15. A method of magnetic resonance imaging using a medical instrument according to claim 12, 13, or 14, wherein the method comprises:
- applying (500) at least one adhesive magnetic resonance imaging antenna (102) to a surface of a subject (418);
- connecting (502) the antenna connector (112) of each of the at least one adhesive magnetic resonance imaging antenna to a preamplifier connector (701) of a preamplifier (422);
- connecting (504) the preamplifier to the magnetic resonance imaging system;
- placing (506) the subject in an imaging zone (408) of the magnetic resonance imaging system;
- controlling (508) the magnetic resonance imaging system with pulse sequence commands (442); and
- receiving (510) magnetic resonance imaging data (444) from the magnetic resonance antenna preamplifier during controlling the magnetic resonance imaging system with the pulse sequence commands; and
- reconstructing (512) a magnetic resonance image (446) from the magnetic resonance imaging data.
